# EUROPÄISCHE PATENTSCHRIFT

(11) **EP 0 611 825 B1**
(45) Veröffentlichungstag und Bekanntmachung des Hinweises auf die Patenterteilung: **13.06.2001**
(21) Anmeldenummer: 94101158.7
(22) Anmeldetag: 27.01.1994
(51) Int. Cl.: C12P 41/00

(54) **Enzymatische Racematspaltung asymmetrischer Alkinole**
Enzymatic cleavage of racemates of asymmetric alkynols
Dédoublement enzymatique de racémates d'alcynols asymétriques

(30) Priorität: 19.02.1993 AT 31993
(43) Veröffentlichungstag der Anmeldung: 24.08.1994
(73) Patentinhaber: DSM Fine Chemicals Austria Nfg GmbH & Co KG, 4021 Linz (AT)
(72) Erfinder: Mayrhofer, Herbert, A-4210 Engerwitzdorf (AT); Wirth, Irma, A-4470 Enns (AT); Pöchlauer, Peter, Dr., A-4020 Linz (AT)
(74) Vertreter: Klostermann, Ingrid

(56) Entgegenhaltungen:
- EP-A- 0 290 878
- EP-A- 0 321 918

## Beschreibung

Optisch aktive Alkinole sind wichtige Zwischenprodukte für die Herstellung von beispielsweise Pheromonen oder pharmazeutisch wirksamen Verbindungen. Für die biologische oder pharmazeutische Wirkung ist dabei die sterische Konfiguration an jenem C-Atom des Alkinols, das die Hydroxylgruppe trägt, ausschlaggebend, da meist nur eine der beiden möglichen Konfigurationen die gewünschte Wirkung entfaltet. Daher ist die Reindarstellung eines der beiden Alkinolenantiomeren von entscheidender Bedeutung.

Verfahren zur enzymatischen Racematspaltung von asymmetrischen Alkinolen sind bereits bekannt. So wird gemäß Chemistry and Physics of Lipids, 54 (1990), 43-48 ein asymmetrisches Alkinol erst mit einer Carbonsäure verestert, worauf das enstandene racemische Estergemisch in Gegenwart einer Lipase enantioselektiv hydrolysiert wird. Bei diesem Verfahren muß die gesamte Mischung aber erst chemisch verestert werden, bevor die enzymatische Enantiomerentrennung erfolgen kann.
In der EP-A-0 290 878 ist geoffenbart, ein Enantiomerengemisch von (R)- und (S)-1-Trimethylsilyl-1-butin-3-ol durch enantioselektive Veresterung mit einem Glycerintriester in Gegenwart einer Hydrolase in den entsprechenden (S)-Alkohol und den entsprechenden (R)-Ester überzuführen, wodurch eine Abtrennung der Enantiomeren voneinander erreicht werden kann. Bei dieser Umsetzung werden aber Alkoholgruppen des Glycerintriesters frei. Da Hydrolasen Substrate in einer Gleichgewichtsreaktion umestern, kann es zur Wiederveresterung dieser Alkoholgruppen kommen, wodurch die Veresterung des Alkinols unvollständig erfolgt. Um diese Wiederveresterung zu unterbinden, ist in J. Am. Chem. Soc. 1990, 112, 7434-7436 vorgeschlagen, für die Racemattrennung von (R,S)-Alkinolen Vinylacetat einzusetzen. Der im Verlauf der Reaktion entstehende Vinylalkohol tautomerisiert zu Acetaldehyd, der die Reaktionsgeschwindigkeit nicht negativ beeinflußt. Dieses Verfahren ist jedoch auf Alkohole beschränkt, die am asymmetrischen C-Atom einen kleinen und einen relativ großen Substituenten tragen, da nur in diesen Fällen annehmbare Selektivitäten erzielt werden können.
In der EP-A-0 321 918 ist ein Verfahren zur enzymatischen Racematspaltung von Alkoholen in Gegenwart einer Lipase und in Gegenwart von Vinylacetat oder Vinylchloracetat als Veresterungsmittel geoffenbart. In einem Beispiel wird die Racematspaltung von (R,S)-1-Octin-3-ol geoffenbart, wobei aber nur sehr unbefriedigende Selektivitäten erreicht werden konnten.

Es wurde nun gefunden, daß bei Verwendung eines Vinylesters, in dem die Säurekomponente mindestens 4 C-Atome aufweist, weitaus höhere Selektivitäten erzielt werden können als mit Vinylacetat, wobei es nicht nötig ist, daß das asymmetrische C-Atom eine großen und einen kleinen Substituenten trägt.

Gegenstand der Erfindung ist daher ein Verfahren zur Erhöhung der Enantioselektivität in einem enzymatischen Verfahren zur Trennung der (R)- und (S)-Enantiomere eines asymmetrischen Alkinols der allgemeinen Formel

R - C ≡ C - CH(OH) - R₁, I

in der R und R₁ unabhängig voneinander Alkylgruppen mit 1 bis 10 C-Atomen und R zusätzliche Wasserstoff bedeuten, in Gegenwart einer Lipase und in Gegenwart eines Vinylesters als Veresterungsmittel, das dadurch gekennzeichnet ist, daß als Veresterungsmittel mindestens ein Vinylester der allgemeinen Formel

CH₂ = CH - O - CO - R₂, II

in der R₂ eine Alkylgruppe mit mindestens 3 C-Atomen bedeutet, eingesetzt wird, dem eine katalytische Menge eines Vinylesters der Formel II, der in der Alkylkette um mindestens zwei C-Atome weniger aufweist, als jener Vinylester, der als Veresterungsmittel eingesetzt wird, zugesetzt wird.

Unter einem asymmetrischen Alkinol ist ein Alkinol mit einem C-Atom, das durch ein Wasserstoffatom, durch eine Alkylgruppe, durch eine Alk-1-inylgruppe und durch eine Hydroxylgruppe substituiert ist, zu verstehen.
In der allgemeinen Formel I bedeuten R und R₁ Alkylgruppen mit 1 bis 10, bevorzugt mit 1 bis 8 C-Atomen, wobei R zusätzlich Wasserstoff bedeutet. Bevorzugt sind die Alkylgruppen geradkettig, beispielsweise Methyl-, Ethyl-, n-Butyl-, n-Octylgruppen. Verbindungen in denen R Wasserstoff und R₁ eine Alkylgruppe mit 1 bis 4 C-Atomen bedeuten, sind besonders bevorzugt. Solche Alkinole sind bekannt und/oder durch bekannte Methoden herstellbar. Das asymmetrische Alkinol kann dabei als racemisches Gemisch oder als Gemisch, in dem eines der beiden Enantiomeren angereichert ist, vorliegen.

In der allgemeinen Formel II bedeutet R₂ eine Alkylgruppe mit mindestens 3, bevorzugt mit 3 bis 15, besonders bevorzugt mit 3 bis 11 C-Atomen, die bevorzugt geradkettig ist. Ganz besonders bevorzugt sind Vinylbutyrat, Vinyloctanoat, Vinyllaurat. Vinylester sind käuflich oder durch bekannte Methoden herstellbar.

Der Unterschied in der Alkylkette des Vinylesters der Formel II und dem in katalytischer Menge zugesetztem Vinylester muß mindestens zwei C-Atome betragen, damit ein wirksamer, enantioselektivitätssteigernder Effekt erreicht wird. Bevorzugt beträgt der Unterschied in der Alkylkette aber mindestens 4, besonders bevorzugt 6 bis 10 C-Atome. Als besonders günstig hat sich ein katalytischer Zusatz von Vinylbutyrat zu Vinylestern der allgemeinen Formel II, in denen R₂ mindestens 8, bevorzugt mindestens 10 Alkylgruppen bedeutet, herausgestellt. Unter katalytischen Mengen sind dabei Mengen zu verstehen, die für eine Trennung des racemischen Gemisches in keiner Weise ausreichen. Bevorzugt wird eine Menge von 0,1 bis 5, besonders bevorzugt von 0,5 bis 4 Gew.% des katalytisch eingesetzten Vinylesters bezogen auf das Gewicht des als Veresterungsmittels eingesetzten Vinylesters der Formel II, zugesetzt.
Unter Lipasen sind zur Racemattrennung geeignete Lipasen zu verstehen, wobei Lipasen aus Schweineleber, Schweinepankreas sowie aus Mikroorganismen, wie Candida, Mucor, Rhizopus, Penicillium, Aspergillus, Pseudomonas bevorzugt sind. Besonders bevorzugt sind käufliche Lipasen, ganz besonders bevorzugt Lipasen aus Candida oder Pseudomonas. Die Lipase kann dabei in gereinigter oder teilweise gereinigter Form oder in Form des Mikroorganismus selbst, in freier oder in immobilisierter Form, bevorzugt in freier Form, eingesetzt werden.

Die Durchführung des erfindungsgemäßen Verfahrens kann nach einer der in der EP-A-0 321 918 geoffenbarten Verfahrensweisen erfolgen. Dazu wird eine Lipase und ein Alkinol der Formel I zusammen mit dem Vinylester der Formel II vorgelegt. Pro Mol Alkinol wird dabei mindestens ein halbes Mol Vinylester eingesetzt. Im allgemeinen wird aber der Vinylester nicht nur als Reaktant, sondern gleichzeitig auch als Lösungsmittel und daher in einem Überschuß eingesetzt. Langkettige Vinylester der Formel II sind aber oft sehr viskos. Zu hohe Viskosität der Reaktionsmischung hat aber einen negativen Effekt auf die Reaktionsgeschwindigkeit. Der Überschuß des Vinylesters in bezug auf das Alkinol wird daher nur so groß gewählt, daß die Mischung nicht so viskos wird, daß die Reaktionsgeschwindigkeit sinkt. Die nötige Menge der Lipase ist von der chemischen Beschaffenheit des Alkinols und des Vinylesters, von der gewünschten Reaktionszeit und von der Art der Lipase abhängig und kann für jeden Fall durch einen Vorversuch leicht ermittelt werden.

Die Reaktionsmischung wird zusammen mit der Lipase bei Temperaturen von -10 °C bis zur Desaktivierungstemperatur der eingesetzten Lipase, bevorzugt bei jener Temperatur, bei der die Lipase die höchste Aktivität besitzt und die vom Hersteller im allgemeinen angegeben ist, vorteilhafterweise gerührt oder geschüttelt. Es ist aber auch möglich, die Lipase in einem Modul, beispielsweise in einer Säule, vorzulegen und die Mischung, die das Alkinol und den Vinylester enthält, im Kreislauf durch dieses Modul zu leiten. Dabei wird eines der beiden Alkinolenantiomeren bevorzugt in den Ester übergeführt, während das zweite Enantiomer als Alkohol unverändert verbleibt. Aus dem verbrauchten Vinylester entsteht der entsprechende Vinylakohol, der zum entsprechenden Aldehyd tautomerisiert.

Der Fortgang der Reaktion wird durch übliche Methoden, beispielsweise durch Gaschromatographie verfolgt. Da eine Lipase zwar bevorzugt eines der beiden Enantiomeren, aber im allgemeinen auch das zweite Enantiomer umsetzen kann, wird in passenden Abständen der Enantiomerenüberschuß ee des nicht umgesetzten Alkohols oder des gebildeten Esters mit Hilfe geeigneter Methoden, beispielsweise durch Bestimmung der optischen Drehung oder durch Chromatographie an einer chiralen Phase, gemessen. Nach Erreichen eines gewünschten Umsetzungsgrades, der vom gewünschten Enantiomerenüberschuß abhängig ist, wird die Reaktion abgebrochen. Zur Aufarbeitung des Reaktionsgemisches wird die Lipase gegebenenfalls beispielsweise durch Abfiltrieren oder Abzentrifugieren aus dem Reaktionsgemisch abgetrennt und der Rückstand einer Trennungsoperation wie etwa Extraktion, Destillation oder Chromatographie unterworfen. Bevorzugt ist dabei eine Destillation, die im allgemeinen besonders wirksam ist, da sich gezeigt hat, daß sich die Siedepunkte des Alkohols, Esters, Vinylesters und des aus dem Vinylester gebildeten Aldehyds für eine einfache, destillative, hochwirksame Trennung genügend unterscheiden. Wird jenes Enantiomer gewünscht, das im Verlauf der Reaktion zum Ester umgesetzt wurde, kann nach Isolierung dieses Esters aus dem Reaktionsgemisch eine Esterspaltung nach üblichen Methoden durchgeführt werden, wobei das gewünschte Enantiomer zumindest angereichert erhalten wird.

Es hat sich unerwarteterweise gezeigt, daß der Zusatz eines organischen Lösungsmittels zur Reaktionsmischung sowohl die Reaktionsgeschwindigkeit, als auch die Stereoselektivität der Reaktion positiv beeinflußt. In einer bevorzugten Ausführungsform wird daher der Reaktionsmischung ein organisches Lösungsmittel zugesetzt. In diesem Fall werden pro Mol Alkinol der Formel I nur 0,5 bis 1,5 Mol, bevorzugt 1,0 bis 1,3 Mol Vinylester der Formel II verwendet. Ein Vorteil des erfindungsgemäßen Verfahrens liegt also darin, daß der Vinylester der Formel II nur als Reaktant und nicht auch als Lösungsmittel eingesetzt werden muß, sodaß ein Teil des Vinylesters durch ein leichter zugängliches organisches Lösungsmittel ersetzt werden kann. Überdies wird durch den Zusatz eines organischen Lösungsmittels eine viskose Reaktionsmischung, die unerwünscht ist, vermieden. Als organisches Lösungsmittel eignen sich gegebenenfalls halogenierte aliphatische oder aromatische Kohlenwasserstoffe wie z.B. Pentan, Hexan, Cyclopentan, Toluol, Xylole, Methylenchlorid, Dichlorethan, Chlorbenzole, Ether, wie z.B. Diethylether, Tetrahydrofuran, Dioxan, Ester, wie z. B. Essigsäureethyl-, -butylester oder Mischungen aus solchen Lösungsmittel, wobei halogenierte aliphatische Kohlenwasserstoffe oder aromatische Kohlenwasserstoffe bevorzugt sind. Das organische Lösungsmittel wird in Mengen von etwa 0,1 bis 70 Vol.% bevozugt von etwa 0,5 bis 60 Vol.% bezogen auf die gesamte Reaktionsmischung zugesetzt.

Es wurde weiterhin unerwarteterweise gefunden, daß der Zusatz von Wasser zur Reaktionsmischung in einer Menge von etwa 0,05 bis 0,5 Vol.%, bevorzugt von 0,1 bis 0,3 Vol.% bezogen auf die gesamte Reaktionsmischung, die Reaktionsgeschwindigkeit erhöht. In einer bevorzugten Ausführungsform werden daher der Reaktionsmischung von 0,05 bis 0,5 Vol.% Wasser bezogen auf die gesamte Reaktionsmischung zugesetzt.

In einer ganz besonders bevorzugten Ausführungsform der Erfindung werden mit einem Mol 3-Butin-2-ol 1,0 bis 1,5 Mol Vinylbutyrat oder Vinyllaurat mit einem Gehalt von 0,1 bis 5 Gew.% Vinylbutyrat, 60 Vol.% Dichlormethan und 0,3 Vol.% Wasser, jeweils bezogen auf die gesamte Reaktionsmischung, vermischt und bei Temperaturen von 15 bis 50 °C über ein Modul, das mit einer Pseudomonas - oder Candida - Lipase befüllt ist, im Kreislauf gepumpt. Die Reaktion wird bei einem gewünschten Umsetzungsgrad, der wie oben beschrieben bestimmt wird, abgebrochen und das Reaktionsgemisch fraktioniert destilliert.

Mit Hilfe des erfindungsgemäßen Verfahrens wird ein Gemisch aus einem (R)- und einem (S)-Alkinol mit hoher Selektivität in ein Gemisch, das das eine Enantiomer in Form des Alkohols und das andere in Form des Esters enthält, übergeführt, worauf die beiden Enantiomere durch eine einfache, physikalische Trennoperation getrennt werden können. Das Verfahren stellt daher eine Bereicherung der Technik dar.

### Beispiel 1

Eine Mischung aus 50 g (R,S)-3-Butin-2-ol (0,713 Mol), 209,8 g Vinyllaurat (0,927 Mol), 460 ml Dichlormethan und 0,3 Vol.% Wasser, bezogen auf die gesamte Reaktionsmischung, wurden bei einer Temperatur von 30 °C über ein Modul, das mit 10 g Pseudomonas - Lipase Amano PS befüllt war, im Kreislauf gepumpt. Der Fortgang der Reaktion und der Enantiomerenüberschuß ee des (S)-3-Butin-2-ols wurde gaschromatographisch bestimmt. Nach 119 Stunden war ein Umsatz von 80 % und ein ee des nicht umgesetzten (S)-3-Butin-2-ols von 91 % erreicht.
Die Mutterlauge wurde im Vakuum destilliert, und durch den Destillationssumpf wurde Stickstoff geleitet, um weiteres 3-Butin-2-ol auszutreiben. Alle erhaltene Destillate, die das 3-Butin-2-ol enthielten, wurde anschließend über eine Vigreuxkolonne bei Normaldruck rektifiziert. Die Hauptfraktion wurde bei einer Kopftemperatur von 104 - 108 °C abgenommen. Dabei wurden 4,8 g (S)-3-Butin-2-ol, das sind 48 % des nicht umgesetzten (R,S)-3-Butin-2-ols mit einem Enantiomerenüberschuß ee von 91 % erhalten.

### Beispiel 2

3,8 g (R,S)-3-Butin-2-ol (54,2 mMol), 45 ml Dichlormethan, 0,3 ml Wasser und 0,8 g Pseudomonas - Lipase Amano P wurden eine halbe Stunde gerührt, mit 12 g Vinyloctanoat (70,5 mMol) versetzt und bei 40 °C 70 Stunden geschüttelt, wobei ein Umsatz von 80 % erreicht wurde. Der Enantiomerenüberschuß des nicht umgesetzten (S)-3-Butin-2-ol betrug 96 %. Nach Abfiltrieren des Enzyms über Celite wurde die Mutterlauge destilliert.

Dabei wurden 0,36 g (S)-3-Butin-2-ol, das sind 47 % des nicht umgesetzten (R,S)-3-Butin-2-ols mit einem Enantiomerenüberschuß ee von 96 % erhalten.

### Beispiel 3

7,13 mMol eines racemischen Alkinols wurden mit 0,02 ml Wasser, 9,3 mMol Vinylester, 6,0 ml Dichlormethan und 0,1 g Lipase Amano PS vermischt und bei 37 °C geschüttelt. Der Fortgang der Reaktion wurde mit Hilfe von Gaschromatographie an einer Cyclodextrinsäule kontrolliert. Nach etwa 20 Stunden wurde die Reaktion abgebrochen. Dabei wurden die in Tabelle 1 zusammengefaßten Ergebnisse erhalten:

**Tabelle 1**

| Alkinol | Vinylester | ee Alkohol | ee Ester |
|---|---|---|---|
| 1-Pentin-3-ol | Vinylacetat | 29 % | 43 % |
| 1-Pentin-3-ol | Vinylbutyrat | 50 % | 28 % |
| 1-Pentin-3-ol | Vinyllaurat | 61 % | 86 % |

In der Tabelle 1 bedeuten:
- ee Alkohol:: Enantiomerenüberschuß des nicht umgesetzten (S)-Alkinols
- ee Ester:: Enantiomerenüberschuß des unter Enzymeinfluß entstandenen (R)-Alkinylesters

### Beispiel 4

0,5 g (R,S)-1-Octin-3-ol (4,0 mMol) wurden mit 0,025 ml Wasser, 0,59 g Vinylbutyrat (5,15 mMol), 4,5 ml Dichlormethan und 0,1 g Lipase Amano PS vermischt und bei 37 °C geschüttelt. Nach 17 Stunden wurden (S)-1-Octin-3-ol mit einem Enantiomerenüberschuß ee von 55 % und (R)-Buttersäure-1-octin-3-ylester mit einem Enantiomerenüberschuß ee von 71 % erhalten.

### Beispiel 5

5 g (R,S)-3-Butin-2-ol wurden mit 10,6 g Vinylbutyrat, 60 ml Dichlormethan und 1,0 g Pseudomonas - Lipase Amano P vermischt und bei 40 °C am Schüttler inkubiert. Nach 195 Stunden war ein Umsatz von 47 % erreicht. Das nicht umgesetzte (S)-3-Butin-2-ol wies einen Enantiomerenüberschuß ee von 49 % auf.
Auf die beschriebene Art und Weise, aber unter Zusatz von 0,4 ml Wasser (0,5 % des Gesamtvolumens der Reaktionsmischung), wurde nach 92,5 Stunden ein Umsatz von 78 % erreicht. Das nicht umgesetzte (S)-3-Butin-2-ol wies einen Enantiomerenüberschuß ee von 93 % auf.

### Beispiel 6

Auf die im Beispiel 3 beschriebene Art und Weise, aber unter Verwendung von Vinyllaurat anstatt Vinylbutyrat, wurde nach 74 Stunden das nicht umgesetzte (S)-3-Butin-2-ol mit einem Enantiomerenüberschuß von 70 % erhalten. Auf dieselbe Art und Weise, aber unter Verwendung eines Gemisches aus Vinyllaurat und 1,7 Mol% Vinylbutyrat (bezogen auf Vinyllaurat), anstatt Vinyllaurat, wurde nach 89,5 Stunden das nicht umgesetzte (S)-3-Butin-2-ol mit einem Enantiomerenüberschuß ee von > 98 % erhalten.

### Beispiel 7

10 g R,S-3-Butin-2-ol (0,143 Mol) wurden mit 42 g Vinyllaurat (0,186 Mol), 90 ml o-Xylol, 0,4 ml Vinylbutyrat und 0,4 ml Wasser vermischt und bei 40°C über ein Enzymmodul, welches mit einer Mischung aus 6 g Pseudomonas Amano PS Lipase und 6 g Celite befüllt war, im Kreislauf gepumpt. Nach 5,5 Stunden wurde die Reaktion abgebrochen. Aus dem Reaktionsgemisch wurde S-3-Butino-2-ol mit einem Enantiomerenüberschuß ee von 95 % isoliert.

### Beispiel 8

Wurde wie Beispiel 7, aber unter Zugabe eines Enzymimmobilisats, gewonnen durch adsorptive Bindung von 6 g Pseudomonas Amano PS Lipase an einen Kunststoffträger (EP100), in die Reaktionsmischung, anstatt Pumpens der Reaktionsmischung über ein enzymgefülltes Modul, ausgeführt. Nach 21 Stunden Schütteln der Reaktionsmischung bei 40°C wurde S-3-Butin-2-ol mit einem Enantiomerenüberschuß ee von 90 % gewonnen.

### Beispiel 9

1 g R,S-3-Butin-2-ol wurde mit 4,2 g Vinyllaurat, 9 ml o-Xylol, 0,04 ml Vinylbutyrat und 0,01 ml Wasser vermischt und mit 6 g Novo-Enzym SP 524 und 6 g Celite bei 40 °C geschüttelt. Das nicht umgesetzte S-3-Butin-2-ol wies nach 28 Stunden einen Enantiomerenüberschuß ee von 63 % auf.

Umsatz und Enantiomerenüberschuß wurden gaschromatographisch, der Enantiomerenüberschuß dabei mit Hilfe von Cyclodextrin, einem chiralen Säulenmaterial, bestimmt.

## Patentansprüche

1. Verfahren zur Erhöhung der Enantioselektivität in einem enzymatisches Verfahren zur Trennung der (R)- und (S)-Enantiomere eines asymmetrischen Alkinols der allgemeinen Formel
R - C ≡ C - CH(OH) - R₁, I
in der R und R₁ unabhängig voneinander Alkylgruppen mit 1 bis 10 C-Atomen und R zusätzlich Wasserstoff bedeuten, in Gegenwart einer Lipase und in Gegenwart eines Vinylesters als Veresterungsmittel, dadurch gekennzeichnet, daß als Veresterungsmittel mindestens ein Vinylester der allgemeinen Formel
CH₂ = CH - O - CO - R₂, II
in der R₂ eine Alkylgruppe mit mindestens 3 C-Atomen bedeutet, eingesetzt wird, dem eine katalytische Menge eines Vinylesters der Formel II, der in der Alkylkette um mindestens zwei C-Atome weniger aufweist, als jener Vinylester, der als Veresterungsmittel eingesetzt wird, zugesetzt wird.

2. Verfahren nach Anspruch 1, dadurch gekennzeichnet, daß der Reaktionsmischung ein organisches Lösungsmittel in einer Menge von 0,1 bis 60 Vol.%, bezogen auf die gesamte Reaktionsmischung, zugesetzt wird.

3. Verfahren nach einem der Ansprüche 1 oder 2, dadurch gekennzeichnet, daß als organisches Lösungsmittel ein aliphatischer, halogenierter Kohlenwasserstoff oder ein aromatischer Kohlenwasserstoff eingesetzt wird.

4. Verfahren nach einem der Ansprüche 1 bis 3, dadurch gekennzeichnet, daß der Reaktionsmischung Wasser in einer Menge von 0,05 bis 0,5 Vol.%, bezogen auf die gesamte Reaktionsmischung, zugesetzt wird.

5. Verfahren nach einem der Ansprüche 1 bis 4, dadurch gekennzeichnet, daß eine Verbindung der allgemeinen Formel I nach Anspruch 1, in der R und R₁ unabhängig voneinander eine Alkylgruppe mit 1 bis 8 C-Atomen und R zusätzlich Wasserstoff bedeuten, eingesetzt wird.

6. Verfahren nach einem der Ansprüche 1 bis 5, dadurch gekennzeichnet, daß eine Verbindung der allgemeinen Formel I nach Anspruch 1, in der R Wasserstoff bedeutet, eingesetzt wird.

7. Verfahren nach einem der Ansprüche 1 bis 6, dadurch gekennzeichnet, daß eine Verbindung der allgemeinen Formel II nach Anspruch 1, in der R₂ eine geradkettige Alkylgruppe mit 3 bis 15 C-Atomen bedeutet, eingesetzt wird.

8. Verfahren nach einem der Ansprüche 1 bis 7, dadurch gekennzeichnet, daß eine Lipase aus einem Mikroorganismus der Gattung Pseudomonas oder Candida eingesetzt wird.

9. Verfahren nach einem der Ansprüche 1 bis 8, dadurch gekennzeichnet, daß das Reaktionsgemisch nach erfolgter Reaktion destillativ aufgetrennt wird.

## Claims

1. Process for enhancing enantioselectivity in an enzymatic process for the separation of the (R)- and (S)-enantiomers of an asymmetric alkynol of the formula
R - C ≡ C - CH(OH) - R₁ I
in which R and R₁ independently of each other denote alkyl groups having 1 to 10 C atoms and in which R additionally denotes hydrogen, in the presence of a lipase and in the presence of a vinyl ester as esterifiying agent, characterized in that the esterifying agent used comprises at least one vinyl ester of the formula
CH₂ = CH - O - CO - R₂ II
in which R₂ denotes an alkyl group having at least 3 C atoms, to which is added a catalytic amount of a vinyl ester of the formula II which has at least two C atoms fewer in the alkyl chain than that vinyl ester which is used as esterifying agent.

2. Process according to Claim 1, characterized in that an organic solvent is added to the reaction mixture in an amount of 0.1 to 60 vol. % based on the entire reaction mixture.

3. Process according to Claim 1 or 2, characterized in that the organic solvent used is an aliphatic, halogenated hydrocarbon or an aromatic hydrocarbon.

4. Process according to one of Claims 1 to 3, characterized in that water is added to the reaction mixture in an amount of 0.05 to 0.5 vol. % based on the entire reaction mixture.

5. Process according to one of Claims 1 to 4, characterized by the use of a compound of the formula I according to Claim 1 where R and R₁ independently of each other denote an alkyl group having 1 to 8 C atoms and R additionally denotes hydrogen.

6. Process according to one of Claims 1 to 5, characterized by the use of a compound of the formula I according to Claim 1 where R denotes hydrogen.

7. Process according to one of Claims 1 to 6, characterized by the use of a compound of the formula II according to Claim 1 where R₂ denotes a straight-chain alkyl group having 3 to 15 C atoms.

8. Process according to one of Claims 1 to 7, characterized in that a lipase from a microorganism of the genus Pseudomonas or Candida is used.

9. Process according to one of Claims 1 to 8, characterized in that the reaction mixture is separated by distillation after the reaction has ended.

## Revendications

1. Procédé destiné à l'augmentation de l'énantiosélectivité dans un procédé enzymatique de séparation des énantiomères (R) et (S) d'un alcynol asymétrique de la formule générale
R - C ≡ C - CH(OH) - R₁, I
dans laquelle R et R₁ signifient indépendamment l'un de l'autre des groupes alkyles avec 1 à 10 atomes de C, et R signifie en plus de l'hydrogène, en présence d'une lipase et en présence d'un ester de vinyle en tant qu'agent d'estérification, caractérisé en ce que, comme agent d'estérification, il est utilisé au moins un ester de vinyle de la formule générale
CH₂ ≡ CH - O - CO - R₂, II
dans laquelle R₂ signifie un groupe alkyle avec au moins 3 atomes de C, auquel est ajouté une quantité catalytique d'un ester de vinyle de la formule II, qui contient dans la chaîne alkyle au moins deux atomes de C de moins que l'ester de vinyle utilisé en tant qu'agent d'estérification.

2. Procédé selon la revendication 1, caractérisé en ce qu'un solvant organique est ajouté au mélange réactionnel en une quantité de 0,1 à 60 % en volume, rapportée à l'ensemble du mélange réactionnel.

3. Procédé selon la revendication 1 ou 2, caractérisé en ce qu'un hydrocarbure halogéné aliphatique ou un hydrocarbure aromatique est utilisé en tant que solvant organique.

4. Procédé selon l'une quelconque des revendications 1 à 3, caractérisé en ce que de l'eau est ajoutée au mélange réactionnel en une quantité de 0,05 à 0,5 % en volume, rapportée à l'ensemble du mélange réactionnel.

5. Procédé selon l'une quelconque des revendications 1 à 4, caractérisé en ce qu'il est utilisé un composé de la formule générale I selon la revendication 1, dans laquelle R et R₁ signifient indépendamment l'un de l'autre un groupe alkyle avec 1 à 8 atomes de C, et R signifie en plus de l'hydrogène.

6. Procédé selon l'une quelconque des revendications 1 à 5, caractérisé en ce qu'il est utilisé un composé de la formule générale I selon la revendication 1, dans laquelle R signifie de l'hydrogène.

7. Procédé selon l'une quelconque des revendications 1 à 6, caractérisé en ce qu'il est utilisé un composé de la formule générale II selon la revendication 1, dans laquelle R₂ signifie un groupe alkyle à chaîne linéaire avec 3 à 15 atomes de C.

8. Procédé selon l'une quelconque des revendications 1 à 7, caractérisé en ce qu'il est utilisé une lipase d'un micro-organisme de l'espèce Pseudomonas ou Candida.

9. Procédé selon l'une quelconque des revendications 1 à 8, caractérisé en ce que, après la réaction, le mélange réactionnel est séparé par distillation.
